# DEMANDE DE BREVET EUROPEEN

(11) **EP 3 381 439 A1**
(43) Date de publication de la demande: **03.10.2018**
(21) Numéro de dépôt: 18165297.5
(22) Date de dépôt: 30.03.2018
(51) Int. Cl.: A61K 8/73, A61Q 19/00

(54) **MASQUE OU PATCH PRÉPARÉ À PARTIR D'UNE COMPOSITION D ALGINATE**

(30) Priorité: 31.03.2017 FR 1752788
(71) Demandeur: Lessonia, 29800 Saint Thonan (FR)
(72) Inventeur: URIEN, Gaëtane, 29440 Saint Derrien (FR); LEBRETON, Stéphane, 29800 Landerneau (FR)
(74) Mandataire: Ipside

(57) **Abrégé**

Produit cosmétique pour la fabrication d'au moins un patch cosmétique, comprenant deux solutions, une solution A et une solution B, la solution B comprenant une phase organique comportant une source de calcium et la solution A comprenant une phase aqueuse comportant un alginate.

## Description

La présente invention est du domaine des patchs et des masques cosmétiques, et concerne en particulier de tels patchs (ou masques) comprenant au moins un alginate. L'invention concerne aussi une composition pour la préparation de tels patchs (ou masques), ainsi qu'un procédé de préparation du patch ou du masque à partir de ladite composition. L'invention concerne encore plus particulièrement les patchs ou masques prêt à l'emploi.

De tels patchs (ou masques) dits prêts à l'emploi sont avantageusement obtenus par coulage dans un récipient (moule) thermoformé de forme voulue de sorte à pouvoir réaliser directement des soins cosmétiques sur la peau (visage, contour des yeux, cou, voire toute autre partie du corps).

Une préparation connue de masques et de patchs cosmétiques est réalisée à partir d'une matrice plane qualifiée de feuille, comprenant au moins une couche à laquelle sont intégrées des ingrédients cosmétiques, et sur laquelle sont pratiquées des découpes adaptées aux contours du visage et/ou de l'une de ses parties (le nez, le coin de l'oeil, le front, etc). La demande FR 2971933 décrit une préparation d'un tel masque comprenant une feuille constituée d'une ou plusieurs couches d'un matériau non tissé. Une telle préparation est difficile à mettre en oeuvre, car elle requiert l'utilisation d'un outillage complexe, et rend inévitable la perte d'importantes quantité de matière (chutes) et donc de matières première.

Il est également connu de préparer des patchs cosmétiques en effectuant un moulage à chaud dans un moule dans laquelle est placé une couche d'un hydrogel contenant des ingrédients cosmétiques. L'hydrogel classiquement utilisé dans une telle préparation est un hydrogel de carraghénanes, lequel implique selon la méthode la plus courante, la difficile réalisation d'un moulage à chaud pour diminuer la viscosité des carraghénanes. Le maintien à chaud complique la mise en oeuvre à l'échelle industrielle, et est peu compatible avec la majorité des conservateurs, lesquels sont détruits à la chaleur mettant en péril la stabilité microbienne des patchs. En outre, le recourt à l'usage de principes actifs thermosensibles est proscrit dans ce contexte. Les autres gélifiants utilisés en complément ou en remplacement des carraghénanes, tels que l'agar agar ou la gomme de caroube, présentent les mêmes inconvénients.

La demande FR 2963888 décrit un masque ou un patch préparé à base d'un hydrogel comportant un carraghénane par choc thermique négatif. Bien qu'il ne soit apparemment pas recouru à l'usage de la chaleur, ce procédé reste compliqué pour une application industrielle de par le nombre important d'étapes nécessaires, lesquelles étapes doivent être étroitement contrôlées en terme de conditions opératoires mises en oeuvre: gammes d'épaisseur, plage de température...

Un autre inconvénient lors de l'emploi des carraghénanes est qu'ils ont une odeur prononcée qui est le plus souvent perçue comme désagréable pour les utilisateurs des masques et des patchs. De plus, parmi d'autres inconvénients connus des masques de carraghénane, l'adhérence médiocre sur la peau humaine est le plus souvent constatée.

Pour la préparation de masques cosmétiques, il est également connu d'avoir recourt à l'utilisation des alginates dans le cadre de la fabrication des masques dits « masques peel-off ». De tels masques peel-off sont remarquables par le fait que la prise en masse doit se faire après un délai de seulement quelques minutes ce qui explique que leurs formulations ne conviennent pas à la fabrication des masques ou patchs cosmétiques sur des périodes de temps assez longue, typiquement de l'ordre d'une journée (car le masque prendrait en masse avant la fin du processus de fabrication). En outre, de tels masques peel-off comportent généralement des agents de charges, en particulier la terre de diatomée, dont l'utilisation est controversée en terme d'hygiène et de sécurité.

D'autres utilisations de l'alginate pour la fabrication de patchs cosmétiques ou dermatologiques sont également connues. Un exemple d'une telle fabrication est décrit dans la demande FR 2784295 qui mentionne le recours à un alginate pour la préparation d'un timbre cutané. Les procédés décrits dans l'art antérieur qui mettent en oeuvre l'utilisation d'alginates ne sont pas satisfaisants, car ils ne permettent pas de maîtriser la durée nécessaire à la gélification des patchs durant la fabrication par coulage à froid.

La présente invention vise à résoudre les problèmes de l'art antérieur et concerne à cet effet un produit cosmétique pour la fabrication d'au moins un patch cosmétique, comprenant deux solutions, une solution A comprenant un alginate et une solution B comprenant une source de calcium ; les solutions A et B étant de préférence immiscibles ou partiellement miscibles. Avantageusement, la solution B comprend une phase organique comportant une source de calcium et la solution A comprend une phase aqueuse comportant un alginate. Plus avantageusement, la solution B comprend une phase organique comportant une source de calcium et une huile et la solution A comprend une phase aqueuse comportant les ingrédients suivants :
- un alginate soluble dans l'eau ;
- un agent de complexation d'au moins un cation de l'alginate ;
- un tensioactif ; et
- une eau
étant entendu qu'un alginate incorpore au moins un cation (tel que du sodium, du potassium, etc).

Dans le cadre de l'invention, le terme « patch » est indifféremment utilisé pour désigner tout support destiné à être posé en surface de la peau pour une utilisation cosmétique (un masque, un patch, etc).

Le produit cosmétique selon l'invention comprend avantageusement :
- de 80 et 99%, et de préférence de 85 à 95%, de la solution A pris en pourcentage massique par rapport à la masse totale dudit produit cosmétique ; et
- de 1 et 20%, et de préférence de 5 et 15%, de la solution B pris en pourcentage massique par rapport à la masse totale dudit produit cosmétique.

Avantageusement, le pH de la phase aqueuse sera compris entre 7 et 10 unités pH, borne comprises.

Un tel produit permet de préparer un patch en réalisant un moulage à froid. Le temps de prise du patch peut être étroitement contrôlé grâce à une composition préparée à partir des deux solutions A et B, et est de préférence de dix heures, voire de plus de dix heures. Un produit permet également de pouvoir conserver un faible taux en microorganismes. Ce produit permet aussi de préparer des patchs sans devoir recourir à l'usage de substances indésirables telles que les terres de diatomée, et il convient pour la préparation de patchs incolores. Les patchs obtenus sont faciles à appliquer et montrent une parfaite adhérence sur la peau.

La description qui suit détaille des aspects avantageux de la présente invention. Toutes les combinaisons des différents ingrédients pris dans les plages de valeurs qui sont décrits dans la suite de la présente description sont également possibles.

L'alginate soluble dans l'eau est avantageusement un alginate métallique, c'est-à-dire un alginate incorporant un cation métallique, et il s'agit avantageusement d'un alginate de métal alcalin (sodium ou potassium) lesquels sont très solubles dans l'eau. Il peut également s'agir d'au moins un des alginates métalliques sélectionnés parmi un alginate de magnésium, un alginate de zinc, de manganèse... mais ces derniers sont moins solubles et sont donc moins appropriés dans le cadre de la présente invention. L'alginate métallique de la phase aqueuse du produit selon la présente invention est avantageusement un alginate de sodium. La teneur en alginate de sodium est avantageusement de 1 à 5 % en pourcentage pondéral par rapport à la masse totale de la phase aqueuse et de préférence elle est de 1.5 à 3.0 %.

En combinaison ou de manière alternative avec ce qui précède, l'alginate soluble dans l'eau est avantageusement un alginate d'ammonium, voire un alginate d'ammonium quaternaire.

L'agent de complexation d'au moins un cation de l'alginate de la phase aqueuse de la solution A est avantageusement un agent chélatant, pouvant également être qualifié de « chélateur », apte à chélater les cations de l'alginate. L'agent chélatant est de préférence un séquestrant du sodium.

Le terme « agent chélatant » au sens de l'invention doit être interprété au sens large et englobe toute molécule apte à se lier sélectivement à un cation ou un groupe de cation, voire à le piéger en fonction de sa taille, et recouvre notamment la notion de cryptands (typiquement les molécules bycliques pontées avec des chaînes carbonées intégrant des hétéroatomes). Un exemple d'agent chélatant connu est l'acide éthylène diamine tétraacétique, bien connu sous l'acronyme « EDTA ».

L'agent de complexation d'au moins un cation de l'alginate est avantageusement un séquestrant du sodium choisi parmi un monophosphate et/ou un polyphosphate. Le monophosphate et/ou le polyphosphate est avantageusement sélectionné parmi au moins l'un des éléments suivants : le tétrasodium de pyrophosphate (Na₄P₂O₇), le trisodium phosphate (Na₃PO₄), le disodium phosphate (Na₂HPO₄) et l'hexamétaphosphate de sodium (NaPO₃)₆. Avantageusement, ledit séquestrant du sodium est du tétrasodium de pyrophosphate (Na₄P₂O₇) pris dans une teneur de 0.01 à 1 % et de préférence elle est de 0.05 à 0.5 % donnée en pourcentage pondéral par rapport à la masse totale de la phase aqueuse. L'agent chélateur a pour fonction la séquestration du cation de l'alginate pour accélérer la réaction d'échange de cation, en vue de déplacer les équilibres chimiques vers la formation d'alginate de calcium, l'apport de calcium étant favorisé par la source de calcium de la solution B.

Le tensioactif peut être choisi parmi les tensioactifs utilisés en cosmétiques. Il peut s'agir de tensio-actifs anioniques (un alkyl benzène sulfate de sodium, un alkyl éther sulfate de sodium, des sels d'acides gras du savon, du sodium coco sulfate...), des tensioactifs cationiques (du type chlorure de benzalkomium), les tensioactifs amphotères (du type alkylpolypeptides, bétaines et alkylimidazolines) et des tensioactifs non ioniques (alkylphénols éthoxylés, alcools éthoxylés, decyl glucoside, alcool cétylique, les glutamates...). Le tensioactif de la phase aqueuse de la solution A est avantageusement pris dans une teneur de 0.05 à 1% et de préférence de 0.1 à 0.8% de la phase aqueuse. Le tensioactif est avantageusement du le dérivé de glycérol et d'acide caprique également connu sous le nom de polyglycéryl 4-caprate (numéro CAS 160391-93-5). Un tel tensioactif permet de favoriser et de stabiliser l'émulsion en vue du mélange des solutions A et B.

L'eau contenue dans la phase aqueuse de la solution A est avantageusement purifiée, elle est de préférence une eau déminéralisée, par exemple par distillation ou par filtration.

La source de calcium de la phase organique de la solution B est de préférence du chlorure de calcium ou du sulfate de calcium. Le sulfate de calcium est préféré de par sa compatibilité à être utilisé en présence de toutes les fonctions chimiques. Avantageusement, la source de calcium est un sulfate de calcium, tel que du dihydrate de sulfate de calcium (gypse), pris dans une teneur de 10 à 20% de la phase organique.

L'huile de la phase organique de la solution B est de préférence une huile à forte teneur en acide gras saturés (c'est-à-dire plus de 70 g d'acide gras saturé pour 100 g d'huile), et de préférence il s'agit d'une huile extraite de noix de coco, connue sous la qualification « d'huile de coco fractionnée » ou encore de « Caprylic Capric Triglycéride ». Lesdit acides gras saturés ont de préférences de 5 à 20 atomes de carbones. Avantageusement, il s'agit d'une huile de coprah à forte teneur en acide gras saturés tels que l'acide caprylique (C₈), l'acide caprique (C₁₀), l'acide laurique (C₁₂) et/ou l'acide myristique (C₁₄). De tels acides gras saturés augmentent la stabilité de la composition car ils retardent les phénomènes d'oxydation. Ces acides gras à chaînes courtes ont par ailleurs l'avantage de conférer un « toucher sec » lié à leur pénétration cutanée facilitée.

La phase aqueuse de la solution A comporte avantageusement un agent humectant tel qu'un glycol, de préférence sélectionné parmi au moins un des glycols suivants : le glycérol, le propylène glycol, le butylène glycol et le pentylène glycol. Avantageusement, l'agent humectant choisi est pris dans une teneur de 2 à 15% de la phase aqueuse. Avantageusement, l'agent humectant choisi est le glycérol dans une teneur de 2 à 9% de la phase aqueuse. Il permet à la fois le dessèchement du patch tout en montrant un effet hydratant pour la peau.

La phase aqueuse de la solution A comporte avantageusement au moins un agent antimicrobien, avantageusement pris dans une teneur de 3 à 6 % de la phase aqueuse, et de préférence de 4 à 5% qui assure la stabilité microbienne de la formule. Ledit agent antimicrobien est avantageusement du pentylène glycol. Un tel agent permet de limiter l'emploi de conservateurs qui ont pour effet d'entraîner des picotements sur la peau. La phase aqueuse comporte avantageusement au moins un deuxième agent antimicrobien, le phénoxyethanol, pris dans un teneur de 0.2 et 0.6%.

La phase aqueuse de la solution A comporte avantageusement un agent d'amélioration métabolique. Un tel agent est au moins une substance bénéfique pour les fonctions métaboliques en particulier de la peau. Il peut s'agir d'une vitamine, d'un oligoélément, de minéraux, d'un acide gras essentiel (par exemple un oméga 3) et/ou un acide aminé essentiel.

La phase aqueuse de la solution A comprend avantageusement les ingrédients suivants dans les plages de valeur données en pourcentage pondéral par rapport à la masse totale de la phase aqueuse :

| | |
|---|---|
| alginate de sodium | 1.5 à 3 % |
| tétrasodium de pyrophosphate | 0.05 à 0.5% |
| polyglycéryl 4-caprate | 0.1 à 0.3% |
| glycérol | 2 à 9 % |
| pentylène glycol | 4 à 5 % |
| actifs cosmétique | 5 à 10 % |

La présente invention concerne également une composition cosmétique obtenue en mélangeant la solution A et la solution B du produit cosmétique pour obtenir une émulsion.

La présente invention concerne également un procédé de préparation de la composition tel que décrite précédemment dans le cadre de l'invention, comprenant les étapes :

La solution B sera ajoutée à la solution A à température ambiante. L'ensemble est agité dans un mélangeur cosmétique (mélangeur rotor-stator) sous vide.

Un tel procédé permet de retarder la prise en masse lente et contrôlée du mélange, grâce à une libération progressive du calcium dans la phase aqueuse à partir de la source de calcium présente dans la phase organique avant le mélange des solutions A et B.

La présente invention concerne aussi une utilisation de la composition selon l'invention pour la préparation d'un patch cosmétique par coulage à froid dans un moule de ladite composition, de préférence à température ambiante. Ledit moule a la forme voulue, il est par exemple choisi pour correspondre sensiblement à l'emprunte de tout ou partie de la pommette d'un utilisateur pour une application en tant que patch anti-cerne, c'est-à-dire sous l'oeil. La présente invention consiste aussi en un tel patch obtenu selon la susdite utilisation de la composition selon l'invention.

Grâce à l'utilisation de la composition selon l'invention, la prise en masse pour obtenir un gel (gélification) est progressive, étant donné la libération progressive du calcium dans la phase aqueuse pour former l'alginate de calcium. Le temps de prise du masque est ainsi maîtrisé et n'intervient pas avant dix heures à partir de la mise en contact des solutions A et B.

Ceci permet la fabrication de patchs sur des périodes de temps d'au moins huit heures. Pour des temps de fabrication qui s'échelonnent sur une journée, il est possible de préparer les solutions A et B en début de journée puis de réaliser le conditionnement dans les moules jusqu'à huit heures après la préparation de la composition. Le moulage peut être réalisé à température ambiante, ce qui implique qu'il n'y a pas de chauffage risquant de détruire les agents de conservation du patch et de ne plus pouvoir garantir un faible taux de microbes.

Avantageusement, la pâte sera conditionné dans des moules thermoformés entre quatre et six heures à compter du mélange des solutions A et B.

De préférence, l'épaisseur du patch est comprise entre 1 et 3 mm.

Les exemples de réalisations qui sont cités à la suite de la présente description ne sont nullement limitatifs, ils sont juste décrits dans un but purement illustratif.

### PARTIE EXPERIMENTALE :

Chacun des ingrédients des solutions A et B est préalablement pesé avec une précision de 0.01 g.

Chacune des solutions A et B est préparée par mélange des ingrédients préalablement pesés dans un mélangeur cosmétique de type rotor-stator, sous vide.

Puis, la solution B est ajouté à la solution A et agité dans le mélangeur cosmétique sous vide.

### Exemple de produit cosmétique constitué d'une solution A et d'une solution B :

### Composition pour patch anti-cernes prévu pour être placé en dessous de l'oeil pour décongestionner cette partie du visage et diminuer la présence des cernes :

Mélange des solutions A et B dans les proportions suivantes :
solution A : 95.3 % et solution B : 4.7 % ;
les solutions A et B étant telles que :
   - **Solution A consiste en la phase aqueuse :**
      Alginate de sodium: 2.10 %
      Glycérol : 5.25 %
      *Antimicrobiens* : pentylène glycol : 4.18 % ;
         phénoxyéthanol : 0.42 % ;
         éthylhexyglycérine : 0.10 %
      Vitamine E : 0.21 %
      *Actifs :* Actif anti-cernes -> un extrait de levure *saccharomyces cerevisiae* : 4.00 % ;
         Actif anti-poches -> un mélange d'hespéridine méthyl chalcone steareth-20, de dipeptide-2 et de palmitoyl Tétrapeptide-7 (commercialisé par la société Sederma sous la référence Eyeliss®) : 2.00 % ;
         Actif anti-irritant -> un extrait de fruit, connu sous la dénomination anglo-saxone « oleyl alcohol Zanthoxylum bungeanum » (commercialisé par la société Indena Spa sous la référence Zanthalene®) : 1.00 %
      *Agent de complexation d'un cation de l'alginate* : tétrasodium de pyrophosphate : 0.30 %
      *Tensioactif :* polyglycéryl-4 Caprate (numéro CAS 160391-93-5) : 0.21 %
      *Autres :*
         Pigment (Oxyde de fer noir) : 2.50 %
         Extrait d'hydroglycérine de réglisse : 0.50%
         Hydrolat d'Hamamelis bio : 0.50 %
         Eau florale de bleuet : 0.50 %
         Caprylic/capric triglycérides (numéro CAS 73398-61-5 / 65381-09-1) : 7.35 %
      Eau : q.s.p. 100 % solution A/phase aqueuse.
   - **Solution B consiste en la phase huileuse :**
      *Source de calcium* : gypse (CaSO₄. 2H₂O) : 14.50 %
      *Huile* : ester triple de glycérol et des acides caprylique et caprique - numéro CAS 73398-61-5 / 65381-09-1 (également qualifié de caprylic/capric triglycérides) : q.s.p. 100% solution B/phase organique.

## Revendications

1. Produit cosmétique pour la fabrication d'au moins un patch cosmétique, comprenant deux solutions, une solution A comprenant une phase aqueuse comportant un alginate et une solution B comprenant une phase organique comportant une source de calcium.

2. Produit cosmétique selon la revendication 1, dans lequel la solution B comprend la phase organique comportant la source de calcium et une huile, et la solution A comprend la phase aqueuse comportant les ingrédients suivants :
- au moins un alginate soluble dans l'eau ;
- un agent de complexation d'au moins un cation de l'alginate ;
- un tensioactif ; et
- une eau.

3. Produit cosmétique selon l'une des revendications 1 ou 2, dans lequel l'alginate est un alginate de sodium pris dans une teneur de 1 à 5 % de la phase aqueuse.

4. Produit cosmétique selon l'une des revendications 1 à 3, dans lequel l'agent de complexation du cation de l'alginate est du tétrasodium de pyrophosphate pris dans une teneur de 0.01 à 1% de la phase aqueuse.

5. Produit cosmétique selon l'une des revendications 1 à 4, dans lequel le tensioactif est pris dans une teneur de 0.05 à 1% de la phase aqueuse.

6. Produit cosmétique selon l'une des revendications 1 à 5, dans lequel la source de calcium est du sulfate de calcium pris dans une teneur de 10 à 20 % de la phase organique.

7. Produit cosmétique selon l'une des revendications 1 à 6, dans lequel l'huile de la phase organique est une huile à forte teneur en acide gras saturés contenant de 5 à 20 atomes de carbones.

8. Produit cosmétique selon l'une des revendications 1 à 7, dans lequel la phase aqueuse comporte du glycérol dans une teneur de 2 à 15% de la phase aqueuse.

9. Produit cosmétique selon l'une des revendications 1 à 8, dans lequel la phase aqueuse comporte au moins un agent antimicrobien pris dans une teneur de 3 à 6 % de la phase aqueuse.

10. Produit cosmétique selon l'une des revendications 1 à 9, dans lequel la phase aqueuse comporte de la vitamine E pris dans une teneur de 0.1 à 0.3 % de la phase aqueuse.

11. Produit cosmétique selon l'une des revendications 1 à 10, dans lequel la phase aqueuse comporte au moins un actif cosmétique.

12. Produit cosmétique selon l'une des revendications 1 à 11, dans lequel la phase aqueuse comprend les ingrédients suivants dans les plages de valeur données en pourcentage pondéral par rapport à la masse totale de la phase aqueuse :
| | |
|---|---|
| alginate de sodium | 1.5 à 3 % |
| tétrasodium de pyrophosphate | 0.05 à 0.5% |
| polyglycéryl 4-caprate | 0.1 à 0.3% |
| glycérol | 2 à 9 % |
| pentylène glycol | 4 à 5 % |
| actifs cosmétique | 5 à 10 % |

13. Composition cosmétique obtenue en mélangeant la solution A et la solution B du produit cosmétique selon l'une des revendications 1 à 12 pour obtenir une émulsion.

14. Patch cosmétique préparé par coulage à froid de la composition selon la revendication 13 dans un moule de la forme voulue.
